# EUROPÄISCHE PATENTSCHRIFT

(11) **EP 1 843 821 B1**
(45) Veröffentlichungstag und Bekanntmachung des Hinweises auf die Patenterteilung: **05.10.2011**
(21) Anmeldenummer: 06706686.0
(22) Anmeldetag: 06.02.2006
(51) Int. Cl.: A61Q 1/10, A61Q 1/00, A61Q 1/04, A61Q 1/08, A61Q 5/00, A61Q 17/04, A61K 8/92, A61K 8/58, A61K 8/34, A61K 8/897

(54) **ZUBEREITUNG, INSBESONDERE KOSMETISCHE ZUBEREITUNG SOWIE DIE HERSTELLUNG UND VERWENDUNG DERSELBEN**
PREPARATION, ESPECIALLY COSMETIC PREPARATION, AND PRODUCTION AND USE THEREOF
PREPARATION, NOTAMMENT PREPARATION COSMETIQUE, PRODUCTION ET UTILISATION DE LADITE PREPARATION

(30) Priorität: 04.02.2005 DE 102005005486
(43) Veröffentlichungstag der Anmeldung: 17.10.2007
(73) Patentinhaber: Schwan-STABILO Cosmetics GmbH & Co. KG, 90562 Heroldsberg (DE)
(72) Erfinder: SWISTOWSKI, Azra, 90491 Nürnberg (DE); ZECH, Christina, 86916 Kaufering (DE)
(74) Vertreter: Leissler-Gerstl, Gabriele
(86) Internationale Anmeldenummer: PCT/EP2006/001031
(87) Internationale Veröffentlichungsnummer: WO 2006/082102

(56) Entgegenhaltungen:
- EP-A- 0 400 546
- EP-A- 0 706 790
- EP-A- 0 979 643
- EP-A- 1 031 342
- EP-A- 1 053 742
- EP-A- 1 559 402
- WO-A-02/41854
- WO-A-2004/108108
- WO-A-2005/097046
- WO-A-2005/107684
- WO-A-2006/013412
- GB-A- 1 140 536
- US-A1- 2003 211 070

## Beschreibung

Die Erfindung betrifft eine Zubereitung insbesondere eine kosmetische Zubereitung, vorzugsweise in flüssiger oder pastöser Form, zur Verwendung auf der Haut, auf Semischleimhäuten, auf Schleimhäuten und auf keratinischen Materialien wie Haaren, Wimpern und Augenbrauen, insbesondere zum Dekorieren, Färben, Verschönen und Pflegen der Haut und der Hautanhangsgebilde. Derartige Zubereitungen finden beispielsweise Verwendung für das Schminken, insbesondere der Wimpern und der Haare - eine solche Zubereitung bezeichnet man dann als "Mascara". Grundsätzlich können die erfindungsgemäßen Zubereitungen bei geeigneter Einstellung und Einfärbung auch als Make-up, Concealer, Camouflage, Lidschatten, Eyeliner, Lipliner, Rouge, Lippenrouge, Lipgloss, Sonnenschutzmittel, Sunblocker, temporäres Tattoo, farbiger Effekt-Sonnenschutz für Surfer und dergleichen verwendet werden. Mascara-Zubereitungen kennt man als sog. "Block-Mascara" oder "Cake-Mascara", eine feste Zubereitungsform, von der die Masse mittels einer angefeuchteten Bürste abgenommen wird, als "Emulsions-Mascara" in Form von O/W- oder W/O-Emulsionen oder in Form einer lösungsmittelbasierten Mascara. Die Verwendung einer "Block-Mascara" wird kompliziert, wenn kein Wasser zum Anfeuchten der Bürste oder der Masse zur Verfügung steht und eine andere geläufige Bezeichnung - nämlich "Spuck-Mascara" - zeigt, dass alternative Anwendungsformen durchaus ernsthafte mikrobiologisch bedingte Gefahren für Produkt und Anwender in sich bergen können.

In einer "Emulsions-Mascara" muß - typbedingt - immer mindestens ein Emulgator verwendet werden, um die kontinuierliche Phase mit der dispergierten Phase in inniger Verbindung zu halten. Nachteilig bei einem solchen Produkt ist meist, dass es nach dem Trocknen auf den Wimpern durch Feuchtigkeit aus der Umgebung, z.B. Wasser, Regen, Schweiß und insbesondere durch die ganz leicht alkalische Tränenflüssigkeit wieder angelöst werden kann, wodurch die Haltbarkeit solcher Zubereitungen verringert ist. Auch der Einsatz von wasserlöslichen oder in Wasser dispergierbaren Polymeren bringt hier nachteilige Effekte. Ein Beispiel für ein solches polymerhaltiges Produkt findet sich bei W. Umbach, "Kosmetik", Georg Thieme Verlag, Stuttgart, 1988, Seite 101. Andere Beispiele finden sich in den Standardwerken der Kosmetik-Literatur, z.B. bei H. Janistyn, "Handbuch der Kosmetika und Riechstoffe", 3. Band, Hüthig-Verlag, 2. Aufl., 1973, Seite 855-860 oder bei G.A. Nowak, "Die kosmetischen Präparate", Verlag Ziolkowsky, 3. Aufl., 1984, 748-751. Erwähnt sei auch eine Emulsions-Mascara auf Polymerbasis, die wenigstens ein Wachs und einen Pseudo-Latex enthält. Um die Emulsion stabil zu halten, muss ein Emulgator enthalten sein. Bekannte konventionelle Emulgatoren sind für die Verwendung oft nachteilig, da sie zu Reizungen führen. Nachteilig bei derartigen Zubereitungen ist ferner, dass sie nach dem Trocknen auf Wimpern oder Haaren einen spröden Überzug bilden, der reißen und abblättern kann. Bei einer Haar-Mascara kann dieser Effekt durchaus erwünscht sein, weil eine solche Zubereitung in Form einer temporären Haarfärbung später durch Ausbürsten wieder leicht entfernbar ist - wenn jedoch feine Teilchen von den Wimpern abblättern, können sie in das Auge gelangen und zu mechanischen Reizen führen oder den Bereich unter dem Auge auch einfärben (sog. "Panda-Bären-Effekt").

Nachteilig bei Emulsionsmascaras ist darüber hinaus, dass sie nach dem Auftragen und Trocknen matt sind. Um die Wimpern besonders zu betonen, ist es jedoch vorteilhaft, wenn die aufgetragene Schicht glänzt.

Bekannt sind auch lösungsmittelhaltige Mascara-Zubereitungen, die nur noch wenig oder gar kein Wasser mehr enthalten. Polare Lösungsmittel wie Ethanol, 1-Propanol, 2-Propanol, Aceton, Methylethylketon, Ethylacetat, Tetrahydrofuran, Dichlormethan und dergleichen schließen sich hierbei aufgrund ihres Geruchs, ihrer stark reizenden Wirkung auf Haut und Schleimhäute und mangelnder Umweltverträglichkeit von selbst aus. Ethanol kann allenfalls in Mengen unterhalb von 15 Vol-% eingesetzt werden. Unpolare Isoparaffine wurden zwar in der Vergangenheit schon verwendet - gegen sie spricht aber bei einigen der Aromatengehalt, was sie für kosmetische Verwendungen ausschließt und die leichte Entflammbarkeit der niedrigsiedenden Isoparaffine, wodurch spezielle Vorkehrungen bei der Produktion derartiger Zubereitungen angeraten sind. Wählt man ausschließlich oder überwiegend höhersiedende C₁₁/C₁₂-Isoparaffine, so erhält man zwar auf Haut und Haaren sehr gut haftende Zubereitungen mit sehr guter Transferresistenz - ihr Geruch wird aber nicht als angenehm empfunden.

Ein Problem bei vielen Mascaras besteht darin, dass sie eine spezielle Verpackung benötigen. Lösungsmittelhaltige Mascaras können nur in solche Behältnisse abgepackt werden, die für das Lösungsmittel inert sind. Wenn das verwendete Lösungsmittel leicht-flüchtig oder nicht immobilisiert ist, muss das Behältnis so dicht sein, dass das Lösungsmittel nicht daraus verdampfen kann. Außerdem ist die Kompatibilität der Kosmetikmasse mit dem Behältnis abhängig von dem jeweils verwendeten Lösungsmittel, so dass für verschiedene Arten von Mascaras unterschiedliche Verpackungen bereitgestellt werden müssen. Dies ist besonders für die Massenproduktion sehr nachteilig.

Aufgabe der Erfindung war es daher, eine Zubereitung insbesondere zum Färben, Verschönen und Pflegen der Haut und der Hautanhangsgebilde zu schaffen, die glänzend ist und auch nach dem Trocknen noch eine glänzende Schicht bilden kann, um z.B. den sogenannten "Wet Look" zu erzeugen. Weiterhin war es Aufgabe der Erfindung, eine Zubereitung zu schaffen, welche sich leicht und gleichmäßig auf Haut, Semischleimhäute, Schleimhäute, Haare und Wimpern auftragen läßt, gut deckt, in einer von den Verwendern als angemessen betrachteten Zeit trocknet und dadurch wischfest wird, lange haftet, nicht in die Feinfältelungen der Haut wandert und sich vom Auftragungsort nicht auf andere Medien, wie z.B. Tassen, Gläser, Metall, Textilien oder andere Hautbezirke und dergleichen überträgt. Hierdurch können unschöne farbige Abdrücke oder Fettfilme entstehen, ölige Bestandteile, die auf der Haut gut spreiten, können nämlich zusammen mit geringen Pigmentmengen migrieren und auf der Haut unschöne Spuren hinterlassen. Produkte die nicht ausreichend auf Haut, Semischleimhäuten, Schleimhäuten, Haaren und Wimpern haften, müssen dann regelmäßig neu aufgetragen werden.

Weiterhin war es eine Aufgabe der Erfindung, eine Zubereitung zu schaffen, die in alle Arten von Behältnissen für kosmetische Massen abgefüllt werden kann, da sie mit den für derartige Behältnisse verwendeten Kunststoffen kompatibel ist. Weiterhin sollte eine Zubereitung zur Verfügung gestellt werden, aus der Lösungsmittel nicht oder nicht ohne weiteres entweichen können, und daher keine speziellen Maßnahmen für die Verpackung erfordern.

Zur Lösung der Aufgabe wird eine Zubereitung vorgeschlagen, wie sie in den Ansprüchen definiert ist. Die erfindungsgemäße Zubereitung hat den Vorteil, dass sie wasserfrei ist und frei ist von O/W- oder W/O-Emulgatoren, und trotzdem eine pastöse Konsistenz hat, so dass sie leicht auf Haare, Wimpern oder Haut aufgetragen werden kann. Überraschenderweise wurde festgestellt, dass eine Kombination aus einer Wachskomponente, wie sie unten definiert wird, einem Silikonfluid als Solubilisierungsmittel und einem Kompatibilisierungsmittel eine Zubereitung ergibt, die die Probleme der Massen, die im Stand der Technik bekannt sind, nicht aufweist, nicht bröselt und sehr haltbare glänzende ästhetische Überzüge liefert.

Ein wesentlicher Bestandteil der erfindungsgemäßen Zubereitung ist die Wachskomponente, die der Zubereitung Struktur gibt. Die Wachskomponente wird aus einem oder mehreren Wachsen und Bestandteilen, die die Viskosität und Strukturierung des/der Wachse beeinflussen, gebildet. Mindestens eines der Wachse ist ein Wachs mit einem Tropfpunkt oberhalb von 60°C, bevorzugt haben, wenn eine Wachsmischung verwendet wird, alle Wachse einen Tropfpunkt über 60°C. Bevorzugt liegt der Tropfpunkt oberhalb von 70°C.

Für die Wachskomponente wird das mindestens eine Wachs ausgewählt aus Wachsen tierischen, pflanzlichen, mineralischen oder synthetischen Ursprungs oder ihren Mischungen und Hybriden. Die verwendeten Wachse weisen eine Härte von 2 bis 40 auf, wobei der Härtewert mit der Methode der Nadelpenetration bestimmt wird. Die Bestimmung erfolgt nach der amerikanischen Norm ASTM D5: Bei einer Temperatur von 25 °C wird eine Nadel mit einem definierten Konus und einem Gewicht von 2,5 g, die mit einem Gewicht von 47,5 g beschwert ist, in eine plane Oberfläche eines Probekörpers eindringen lassen. Bestimmt wird die Eindringtiefe in Zehnteln eines Millimeters nach 5 Sekunden.

Bevorzugt eingesetzt werden Bienenwachs, modifiziertes Bienenwachs oder sog. "Cera Bellina", Lanolinwachs, Japanwachs, Candelillawachs, Ouricuriwachs, Carnaubawachs, Reiswachs, Blütenwachse oder Fruchtwachse wie z.B. Orangenblütenwachs, Orangenwachs, Jasminwachs oder Apfelwachs, Montanwachs, mikrokristallines Wachs, Paraffinwachse, Ozokerit, Polyethylenwachse, nach dem Fischer-Tropsch-Verfahren hergestellte Wachse, Silikonwachse, langkettige Ester wie z.B. Cetylpalmitat, Stearylstearat, Behenylstearat, Behenylbehenat, hydriertes Jojobaöl oder Mischungen daraus und ihre Hybride. Die Einsatzmengen dieser Wachse oder der Mischungen daraus und/oder ihrer Hybride liegen im allgemeinen im Bereich zwischen 3 und 35 Gew.-%, bevorzugt zwischen 5 und 25 Gew.-%, besonders bevorzugt zwischen 8 und 20 Gew.-%, bezogen auf die fertige Zubereitung.

Die Wachskomponente kann weiterhin vorzugsweise mindestens ein nicht flüchtiges ÖI, mindestens einen festen langkettigen Alkohol, und mindestens ein filmbildendes, flexibles Polymer oder Copolymer enthalten. Weitere Inhaltsstoffe, wie Hilfs- und Füllstoffe, Färbemittel, im UVA- und UVB-Bereich wirksame Lichtschutzfilter, Konservierungsmittel, Antioxidantien, Antiabsetzmittel, Riechstoffgemische und dgl. können hinzukommen, wobei Konservierungsmittel in der Regel nicht erforderlich sind. Die erfindungsgemäße Zubereitung ist bevorzugt frei von flüchtigen Mineralölen und von flüssigen oder bei Raumtemperatur festen Triglyceriden sowie von konventionellen Emulgatoren und Wasser.

Um die Wachskomponente noch geschmeidiger zu machen wird mindestens ein nichtflüchtiges ÖI und/oder ein langkettiger Alkohol und/oder ein filmbildendes Polymer oder Copolymer mit einem Tropfpunkt von mindestens 30°C zugefügt. Unter "nicht-flüchtigem ÖI" wird eine hochmolekulare Verbindung auf Basis von Kohlenwasserstoff- oder Siloxaneinheiten verstanden. Bevorzugt als nicht flüchjtiges ÖI sind hochmolekularen Silikone wie Dimethylpolysiloxan (Dimethicone), Phenyltrimethicone, Diphenyldimethicone, und/oder Polybutene und Polyisobutene. Weiterhin können mittel oder langkettige Carbonsäureester wie Jojobaöl, Oleylerucat, Oleyloleat, Diethylsebacat, Hexyllaurat, Dibutyladipat, Isostearyl-isostearat oder Mischungen daraus zugefügt werden. Die Einsatzmenge der nichtflüchtigen ÖIe liegt im allgemeinen zwischen 1 und 30 Gew.-% und bevorzugt zwischen 5 und 15 Gew.-%.

Die Wachskomponente kann weiterhin noch durch einen langkettigen Alkohol mit 14 bis 48, bevorzugt 20 bis 40 Kohlenstoffatomen ergänzt werden. Geeignete langkettige Alkohole werden ausgewählt aus bei Raumtemperatur festen gesättigten geradkettigen oder verzweigtkettigen Fettalkoholen, wie z.B. Myristylalkohol, Cetylalkohol, Stearylalkohol, Behenylalkohol, Cerylalkohol, Myricylalkohol oder Mischungen daraus. Bevorzugt eingesetzt wird ein Gemisch aus Alkoholen einer Kettenlänge zwischen 20 und 40 Kohlenstoffatomen. Die Einsatzmenge der langkettigen Alkohole liegt dabei zwischen 0,5 und 20 Gew.%, vorzugsweise zwischen 1,5 und 15 Gew.-%, besonders bevorzugt zwischen 2 und 12 Gew.%.

Als weiteren Bestandteil, der als Haftvermittler und zur Verbesserung der Abgabe dienen kann, kann die Wachskomponente ein steroidhaltiges Material enthalten. Als steroidhaltiges Material werden Colophonium und seine Derivate, Rosinderivate sowie Sterole und Stanole und deren Derivate angesehen. Als Derivate werden in diesem Zusammenhang ungeladenene Derivate betrachtet, insbesondere Ester mit einwertigen und mehrwertigen Alkoholen und Copolymere; Salze sind hier ausgenommen. In diesem Zusammenhang geeignete Ester sind z. B. die Ester von Rosin acid oder Colophonium mit mehrwertigen Alkoholen, wie Glycerin und Pentaerythrit und Copolymere, die aus mehrwertigen Alkoholen z. B. Glyceryl-Einheiten, Maleinsäure-Einheiten und Rosin acid aufgebaut sind. Zum Einsatz kommen können auch Sterine und Sterinderivate wie Oryzanol bzw. Produkte, die diese Verbindungen enthalten. Beispielhaft zu nennen sind Dipentaerythrityl Hexahydroxystereate/Hexastearate/Hexahydrogenated Rosinate, Dipentaerythrityl Hexahydroxystereate/Hexastearate/Hexarosinate, Glyceryl Dibehenate/Hydrogenated Rosinate, Glyceryl Dimyristate/Hydrogenated Rosinate, Glyceryl Hydrogenated Rosinate, Glyceryl Rosinate, Glyceryl Tri-Hydrogenated Rosinate, Glycol Rosinate, Methyl Hydrogenated Rosinate, Methyl Rosinate, PEG-3 Dirosinate, Pentaerythrityl Hydrogenated Rosinate, Pentaerythrityl Rosinate, TEA-Rosinate, Triethylene Glycol Hrydrogenated Rosinate, und/oder Triethylene Glycol Rosinate; oder Mischungen davon. Geeignet sind auch pflanzliche Extrakte, die sterolartige Verbindungen enthalten, z. B. Rice bran wax. Das Steroidderivat wird in solchen Mengen eingearbeitet, dass es die gewünschte Wirkung erzielt. Bevorzugt wird eine Menge von 0,1 bis 10 Gew. %, besonders bevorzugt 0,5 bis 4 Gew. % eingesetzt. Die Verbindungen können auch weitere vorteilhafte Eigenschaften beitragen, z. B. die Filmbildung fördern und das angenehme Gefühl auf der Haut verstärken.

Um die Wachskomponente zu plastifizieren bzw. pastös zu machen, wird als Solubilisierungsmittel ein Silikonöl eingesetzt, das ein lineares, verzweigtes oder zyklisches Silikon mit 1 bis 20 Siloxaneinheiten, bevorzugt 3 bis 8 Siloxaneinheiten sein kann. Besonders bevorzugt wird das Silikonöl ausgewählt aus linearen kurzkettigen Dimethylsiloxanen mit 2 bis 8, bevorzugt 2 bis 6 Siloxaneinheiten, wie z.B. Hexamethyldisiloxan, Octamethyltrisiloxan, Decamethyltetrasiloxan und/oder Dodecamethylpentasiloxan, oder zyklischen Silikonölen mit 4 bis 7 Siloxaneinheiten, wie z.B. Octamethylcyclotetrasiloxan, Decamethylcyclopentasiloxan, Dodecamethylcyclohexasiloxan oder Mischungen davon. Die Einsatzmenge dieser niedrigmolekularen Silikonöle liegt im Bereich zwischen 10 und 80 Gew.-% bevorzugt zwischen 15 und 65 Gew.-% und ganz bevorzugt zwischen 25 und 50 Gew.-%.

Die erfindungsgemäße Zubereitung ist somit eine Kombination aus einer Wachskomponente und einem Silikonfluid. Um diese wesentlichen Bestandteile zu einer homogen Zusammensetzung zu verarbeiten, wird in einer bevorzugten Ausführungsform ein Kompatibilisierungsmittel zugegeben. Das Kompatibilisierungsmittel ist eine Verbindung, die zwischen der hydrophoben Wachskomponente und dem lipophoben und hydrophoben Silikonfluid vermittelt. Hierzu geeignet sind Alkylpolysiloxane, insbesondere Dimethylsiloxane, die langkettige Alkylreste als Endgruppen und/oder Seitenketten tragen. Bevorzugte Alkylpolysiloxane werden ausgewählt aus Alkyldimethiconen, die Alkylreste unterschiedlicher Kettenlänge tragen können. Die Alkylreste können zwischen 10 und 36 Kohlenstoffatome aufweisen. Sie können zudem auch Hydroxylgruppen aufweisen und/oder über Etherbrücken mit kürzeren Alkylketten verbunden sein. Im weitesten Sinne den Alkyldimethiconen zurechenbar sind Ester der Dimethiconole. Beispielhaft genannt seien hier Stoffe wie Lauryldimethicon, Cetyldimethicon, Cetearyldimethicon, Behenyldimethicon, C24-28 Alkyldimethicon, Bishydroxyethoxypropyldimethicon, Phenylpropylsilsesquisiloxan, Dimethiconolstearat, Hydroxypropyldimethicon-behenat und Gemische daraus. Die Einsatzmenge liegt hierbei zwischen 0,5 und 35 Gew.-%, bevorzugt zwischen 1,5 und 25 Gew.-%, ganz besonders bevorzugt zwischen 3 und 12 Gew.-%.

Die erfindungsgemäße Zubereitung soll nach dem Auftragen gut am aufgetragenen Ort haften und von dort nicht wegwandern. Um die Haftfähigkeit zu verbessern, kann der erfindungsgemäßen Zubereitung ein nicht flüchtiges ÖI oder ein Carbonsäureester zugefügt werden. Beispiele für geeignete nicht flüchtige Silikonöle sind Dimethylpolysiloxane mit einem Molekulargewicht zwischen 50.000 und 1.000.000, bevorzugt 200.000 und 800.000, besonders bevorzugt 300.000 bis 500.000, die mit Phenylgruppen substituiert sein können. Es können z.B. Dimethicone, Phenyltrimethicone oder Diphenyldimethicone mit einem Molekulargewicht im angegebenen Bereich verwendet werden. Beispiele für als Haftmittel geeignete Carbonsäureester sind Jojobaöl, Oleylerucat, Oleyloleat, Diethylsebacat, Hexyllaurat, Dibutyladipat, Isostearylisostearat oder Mischungen daraus. Die Einsatzmenge dieser nicht flüchtigen ÖIe liegt im allgemeinen zwischen 1 und 30 Gew.-% und bevorzugt zwischen 7 und 25 Gew.-%.

Zur Verbesserung der Auftragbarkeit, der Haltbarkeit und der Transferresistenz wird bevorzugt ein filmbildendes flexibles Polymer oder Copolymer eingesetzt, das einen Tropfpunkt von mindestens 30°C aufweist. Bevorzugt wird das Polymer bzw. Copolymer ausgewählt aus Olefin-Maleic Acid-Copolymeren, besonders bevorzugt wird ein langkettiges Alkyl/Isoalkylmaleat/MA Terpolymer verwendet.. Als besonders geeignet haben sich C30-38 Olefin/Isopropyl Maleate/ MA Copolymer, Cetearyl Dimethicone/Vinyldimethicone Crosspolymer, Octadecene/MA Copolymer, Methoxy Amodimethicone Silsesquioxane Copolymer oder Mischungen daraus erweisen. Eingesetzt werden davon zwischen 0,1 und 20 Gew.-%, bevorzugt zwischen 0,5 und 12 Gew.-% und ganz besonders bevorzugt zwischen 2 und 8 Gew.-%.

Die erfindungsgemäße Zubereitung kann darüber hinaus weitere Inhaltsstoffe enthalten, wie sie für kosmetische Zusammensetzungen üblich sind. Diese Inhaltsstoffe dienen dazu, Eigenschaften wie die Viskosität, die Struktur, die Fließfähigkeit und Konsistenz und die Haftfähigkeit des Auftrags, sowie Farbe und ästhetische Parameter einzustellen.

In einer bevorzugten Ausführungsform enthält die erfindungsgemäße Zubereitung zur Einstellung der Konsistenz ein Siliciumdioxid bzw. Silica, insbesondere hochdisperse Kieselsäure mit großer Oberfläche, die das Silikonfluid aufnehmen kann und dadurch zur Kompatibilisierung und Vermeidung der Phasentrennung beiträgt.

Weiterhin können Füllstoffe enthalten sein, wie z.B. Talkum, Kaolin, Heilerde, Smectite, Stärke und modifizierte Stärke, nichtquellende Stärken, Cellulose, Polytetrafluorethylenpulver (Teflon), Polyamidpulver (Nylon), Glimmer, unlösliche Metallseifen wie Mg-Stearat, Ca-Stearat, Sr-Stearat oder Zn-Stearat und/oder anorganische oder organische Pigmente und Verlackungen organischer Farbstoffe. Für letztere seien beispielhaft genannt: Titandioxid, Zinkoxid, gelbes, rotes oder schwarzes Eisenoxid, Chromoxidgrün, Chromoxidhydratgrün, Ultramarin, Berliner Blau (Ferric Blue), Manganviolett, Carmin, Perlglanzpigmente, z.B. mit Titandioxid beschichte Glimmer und farbige mit Titandioxid und Metalloxiden beschichtete Glimmer, Bismuthoxichlorid, mit Metalloxiden beschichtetes Bismuthoxichlorid, plättchenförmige Metallpulver von Aluminium, Bronze, Messing, Kupfer, Silber und Gold, sowie Verlackungen organischer Farbstoffe mit Aluminium, Barium, Calcium, Strontium oder Zirkonium oder Mischungen der genannten Stoffe. Diese Aufzählung ist nur beispielhaft und keinesfalls abschließend. Die Einsatzmengen liegen dabei im üblichen Rahmen und richten sich bevorzugt nach den durch die jeweilige Kosmetik-Gesetzgebung geregelten Höchstmengen. Die Mengenanteile an Füllstoffen und Färbemitteln liegen dabei in einem Bereich zwischen 0,01 und 70 Gew.-%, vorzugsweise in einem Bereich von 1 bis 55 Gew.-% und ganz besonders bevorzugt in einem Bereich von 2 bis 25 Gew.-%.

Vorteilhaft beim Einsatz der vorstehend beispielhaft angeführten Färbemittel in wasserfreien Zubereitungen ist der erzielbare hohe Glanz und die hohe Farbintensität, bedingt durch den Brechungsindex des verwendeten Silikons. Die Zubereitungen sind unabhängig von irgendwelchen durch pH-Werte verursachten Einflüssen, z.B. die Entwicklung von Schwefelwasserstoff bei Verwendung von Ultramarinblau oder eine evtl. Freisetzung von Wasserstoff bei Verwendung von Metallpulvern ist wegen des nichtvorhandenen Wassers als Reaktionspartner ebenfalls nicht zu erwarten. Bei der Verwendung von Silica verwendet man vorteilhaft oberflächenbeschichtete, vorzugsweise hydrophobierte Produkte.

In Sonnenschutzmitteln, insbesondere sog. "Sun-Blockern" können neben öllöslichen Lichtfiltersubstanzen, wie z.B. Isoamyl- oder Ethylhexyl-p-methoxycinnamat oder 4-Methylbenzylidencampfer, auch Kombinationen mit Metalloxiden wie z.B. Titandioxid, Ceroxid, Zinkoxid und Zirkoniumoxid in Form von sog. "Nanopigmenten" mit mittleren Teilchengrößen im Bereich zwischen 5 und 25 nm eingesetzt werden.

Zur Viskositätsregulierung und Stabilisierung der erfindungsgemäßen Zubereitung können durchaus auch an sich bekannte Hilfsstoffe wie Bentonite, Hectorite, Montmorillonite oder auch organisch modifizierte Derivate dieser Hilfsstoffe sowie deren Mischungen eingesetzt werden, die dann ggf. in geeigneter, dem Fachmann bekannter Weise aktiviert werden müssen. Geeignet sind hierzu bekanntermaßen geringe Zusätze von Diethylcarbonat, Propylencarbonat oder alkoholische Lösungen von α-Hydroxysäuren. Natürlich ist es auch möglich, Oleogele unter Verwendung von 12-Hydroxistearinsäure und Behenaten, z.B. Behenamidopropyl Dimethylamine Behenate herzustellen - die entsprechenden Verfahren sind dem Fachmann beispielsweise aus DE 102 01 370 C2 bekannt.

Da die erfindungsgemäßen Zubereitungen wasserfrei sind, kann in der Regel auf den Einsatz von Konservierungsmitteln verzichtet werden. Falls Antioxidantien eingesetzt werden, sind die üblicherweise Verwendeten geeignet. Bevorzugt werden kosmetisch annehmbare Produkte, wie Vitamin-E-acetat eingesetzt.

Die erfindungsgemäßen Zubereitungen liegen in dickflüssiger Form oder in Form geschmeidiger Pasten vor und lassen sich leicht und gleichmäßig auf der Haut, auf Semischleimhäuten, auf Schleimhäuten sowie auf Haaren und Wimpern verteilen. Die Viskosität der erfindungsgemäßen Zubereitungen wird entsprechend dem angestrebten Verwendungszweck eingestellt und liegt im Bereich zwischen 50 und 1.500 mPas bei den flüssigen bis dickflüssigen Zubereitungen und im Bereich zwischen 1,5 und 500 Pas bei den pastösen Zubereitungen. Die Bestimmung der Viskosität erfolgt dabei in konventioneller Weise mit einem handelsüblichen Viskosimeter bei einer Drehzahl von 1 U/min mit einer Platte/Kegel- oder Platte/Platte-Meßeinrichtung, bei letzterer bei einer Spaltbreite von 0,4 mm.

Die erfindungsgemäßen Zubereitungen bilden am Auftragungsort nach dem Abtrocknen eine als angenehm empfundene elastische Schicht, welche sich nicht verschiebt, nicht abfärbt, nicht wandert und den ganzen Tag am Auftragungsort verbleiben kann. Sie kann in den Anwendern bekannter Weise wieder entfernt werden - durch geeignete Abschminkmittel oder -tücher, mittels einer Reinigungslotion oder durch Abwaschen mit Feinseife oder entsprechenden milden Tensidzubereitungen. Ein Vorteil der erfindungsgemäßen Zubereitungen besteht darin, dass sie in jede Art von Behältnis abgefüllt werden können, da sie mit den gängigen, für derartige Behältnisse verwendeten Polymeren kompatibel sind, d.h. mit diesen nicht reagieren und durch diese nicht verändert werden. Da die Zubereitungen keine Lösungsmittel enthalten, die bei der Lagerung unter normalen Bedingungen abgegeben werden, sind spezielle Maßnahmen um ein Entweichen von Lösungsmittel zu verhindern, nicht erforderlich. Die erfindungsgemäßen Zubereitungen können daher in bekannter Weise in für den angestrebten Verwendungszweck geeignete Behältnisse, wie Flaschen mit darin enthaltenen Applikatoren (Spatel, Pinsel, Bürstchen und dergl.), Döschen, Tuben, Tiegel oder Pfännchen (Godets) abgefüllt und daraus vom Verbraucher wieder entnommen werden. Sie können aber auch, wegen der damit verbundenen, verbesserten hygienischen Verhältnisse, in geeignete Auftragvorrichtungen, sog. "Spendermechaniken" eingebracht und daraus appliziert werden. Sogenannte druckbetätigte Dosierspender mit einem nachlaufenden Schleppkolben sind grundsätzlich bekannt. Bekannt ist auch die Möglichkeit, eine Zubereitung als Zweikammer-Aerosol abzufüllen. Für den Auftrag kleiner Mengen, wie sie beispielsweise im Lippen- und Augenbereich benötigt werden, bieten sich Auftragvorrichtungen an, wie sie z.B. aus US 6,238,117 oder US 6,309,128 bekannt sind, da diese eine sehr schöne Feindosierung erlauben.

Werden die erfindungsgemäßen Zubereitungen als "Mascara-Zubereitungen" zum Färben der Wimpern hergestellt, dann sollen sie den Wimpern nicht nur Farbe geben sondern sie auch verstärken, um sie optisch dicker und stärker erscheinen zu lassen und ihnen Schwung zu geben. Üblicherweise erhalten die Zubereitungen dann eine Einfärbung in Schwarz, Braun, Blau und Dunkelgrau. Sofern man lediglich die Naturfarbe von Wimpern verstärken und eindrucksvoller gestalten will, empfiehlt es sich, die Mascara-Zubereitung ohne Füllstoffe und Färbemittel transparent herzustellen. Beabsichtigt man besondere Farbeffekte unter langwelligem UV-Licht (sog. "Schwarzlicht"), um z.B. besondere Effekte unter Discobeleuchtung zu erzielen, kann man einer solchen Mascara-Zubereitung Titandioxid in Form von Nanopigmenten oder für den Einsatz in Kosmetika zugelassene Stilben-Derivate zusetzen. Modisch aktuell ist es, die Spitzen der Wimpern mit kontrastierenden Farben, z.B. Hellgrün, Moosgrün, Gelb, Orange oder Rot, zu akzentuieren. Hierzu bieten sich vorzugsweise die vorgenannten, für den Augenbereich zugelassenen anorganischen Pigmente als Färbemittel an, aber auch z.B. Verlackungen organischer Farbstoffe wie FD&C Blue No. 1 (C.I.-No. 42090), FD&C Red No. 40 (C.I.-No. 16035) oder FD&C Yellow No. 5 (C.I.-No. 19140). Soll die erfindungsgemäße Zubereitung als Haar-Mascara verwendet werden, wird die Farbauswahl naturgemäß vielseitiger, da hierbei dann auch Verlackungen organischer Farbstoffe eingesetzt werden können, die nur für den externen Bereich zugelassen sind. Werden Zubereitungen gewünscht, die unter Discobeleuchtung besondere Farbeffekte ergeben, können die hierzu geeigneten, fluoreszierenden Färbemittel eingesetzt werden, die dem Fachmann hinreichend bekannt sind - siehe hierzu z.B. WO 2004/108108. Möglich ist es auch, in Effekt-Zubereitungen feinteilige Glitzerteilchen aus massegefärbten Polymerträgern wie Polyester, Polycarbonaten oder ähnlichem zu verwenden. Zum Verstärken und Verlängern von Wimpern sind schon länger Mascara-Zubereitungen üblich, die feine Fasern enthalten, z.B. Nylon, Seide, Cellulose, Polyester, Teflon, Rayon und dergleichen.

Behältnisse für Mascara sind handelsüblich und dem Fachmann hinreichend bekannt - er hat hier lediglich zu beachten, dass die gewählte Bürste die Wimpern gut benetzt und dabei vereinzelt und dass Bürste und Abstreifer aufeinander abgestimmt werden. Werden eine Mascara-Zubereitung und eine Effekt-Mascara zusammen angeboten, dann empfiehlt sich eine aus zwei einzelnen Einheiten kombinierbare Verpackung - auch solche Kombi-Verpackungen, bei denen eine Einheit mit geringerem Volumen und einer kleineren Bürste ausgestattet sein kann, sind zwischenzeitlich handelsüblich. Da der Mensch im Normalfall mehr Kopfhaare als Wimpern besitzt, muß für eine Haar-Mascara naturgemäß ein größeres Behältnis gewählt werden.

Die erfindungsgemäße Zubereitung soll nun anhand der nachfolgenden Beispiele im Detail erläutert werden, welche sie jedoch nicht abschließend beschreiben. Die Mengenangaben erfolgen dabei in Gewichtsprozent, bezogen auf das Gesamtgewicht der jeweiligen Zubereitung. Die angeführten Mengenangaben können im Einzelfall leicht über- oder unterschritten werden, wobei dennoch die erfindungsgemäßen Zubereitungen erhalten werden. Dies kommt angesichts der breit streuenden Vielfalt an geeigneten Komponenten derartiger Zubereitungen für den Fachmann keinesfalls unerwartet, so dass er weiß, dass bei solchen Über- oder Unterschreitungen das Gebiet der vorliegenden Erfindung nicht verlassen wird. Zur Kennzeichnung der Rohstoffe werden die dem Fachmann bekannten INCI-Namen verwendet.

### Beispiel 1 (pastenförmige Mascara-Zubereitung)

| | |
|---|---|
| Octamethyltrisiloxan | 35,900 |
| Dimethicone (MG 500.000) | 17,500 |
| Cera Microcristallina (81-84 °C) | 12,000 |
| C24-28 Alkyldimethicone | 8,000 |
| Candelilla Cera | 7,000 |
| C30-38 Olefin/Isopropyl Maleate/MA Copolymer | 6,500 |
| C20-C40 Alcohols | 2,500 |
| Schwarzes Eisenoxid (C.I.-No. 77.499) | 8,000 |
| Silica | 2,000 |
| Tocopherolacetat | 0,350 |
| Fragrance | 0,250 |

Zur Herstellung werden die Rohstoffe der Pos. 2-6 bei etwa 80 °C geschmolzen, dann wird das Färbemittel (schwarzes Eisenoxid) und das Silica zugemischt und die Mischung anschließend dreimal durch einen Dreiwalzenstuhl passiert. Die Mischung wird nun in eine Vakuum-Prozeßanlage überführt, mit Octamethyltrisiloxan, Tocopherolacetat und dem Riechstoffgemisch versetzt, gut durchmischt, unter leichtem Vakuum entlüftet, abgekühlt bis auf etwa 28-30 °C und in Lagerbehältnisse überführt.

Zur Herstellung weiterer Farben werden statt des schwarzen Eisenoxids, Mischungen von schwarzem, rotem und gelbem Eisenoxid zur Herstellung einer braunen Mascara-Zubereitung, Mischungen von schwarzem Eisenoxid mit Titandioxid zur Herstellung einer grauen Mascara-Zubereitung und Ultramarin, ggf. unter Mitverwendung von Titandioxid, zur Herstellung einer blauen Mascara-Zubereitung verwendet. Effektfarben lassen sich durch die Verwendung von gelbem und/oder rotem Eisenoxid, (gelbe, orange, rote Mascara-Zubereitung), von Chromoxidgrün (moosgrüne Mascara-Zubereitung), Chromoxidhydratgrün (türkisfarbene Mascara-Zubereitung), ggf. jeweils auch in Abmischung mit Titandioxid, herstellen. Es liegt dabei im Ermessen des Fachmanns, entsprechend der gewünschten Erfordernisse an Farbton und Farbintensität, Variationen des vorstehenden Beispiels vorzunehmen. Reduzierungen der Pigmentmenge können dabei durch eine Erhöhung des Anteils an flüchtigen Ölen oder durch Zusatz von unbeschichtetem Glimmer oder Talkum ausgeglichen werden.

### Beispiel 2 (pastenförmige, farblose Mascara-Zubereitung)

| | |
|---|---|
| Decamethyltetrasiloxan | 41,600 |
| Dimethicone (MG 350.000) | 12,500 |
| Cera Microcristallina (81-84 °C) | 12,000 |
| C24-28 Alkyldimethicone | 5,500 |
| Carnauba Wax (Copernicia Cerifera) | 6,500 |
| C30-38 Olefin/lsopropyl Maleate/MA Copolymer | 4,500 |
| C20-C40 Alcohols | 4,500 |
| Titandioxid (Nanopigment 10-20 nm) | 8,000 |
| Silica | 2,500 |
| Silica Silylate | 1,800 |
| Tocopherolacetat | 0,350 |
| Fragrance | 0,250 |

Die Herstellung von Beispiel 2 erfolgt in Analogie zu Beispiel 1. Diese Mascara-Zubereitung ergibt auf der Wimper einen fast farblosen und transparenten Überzug, der die Wimper verdickt und leicht verlängert. Unter langwelligem UV-Licht zeigt diese Zubereitung ein intensives hellblaues Leuchten. Um ein auch unter UV-Licht gänzlich farbloses Produkt zu erhalten, ersetzt man das Titandioxid-Nanopigment durch die entsprechende Menge an Decamethyltetrasiloxan.

### Beispiel 3 (pastenförmige Haar-Mascara)

| | |
|---|---|
| Decamethyltetrasiloxan | 37,900 |
| Dimethicone (MG 500.000) | 14,500 |
| Cera Microcristallina (81-84 °C) | 12,000 |
| C24-28 Alkyldimethicone | 5,500 |
| Bienenwachs, weiß | 6,500 |
| C30-38 Olefin/Isopropyl Maleate/MA Copolymer | 4,500 |
| C20-C40 Alcohols | 4,500 |
| Titandioxid | 2,500 |
| Rotes Eisenoxid (C.I.-No. 77.491) | 5,000 |
| Gelbes Eisenoxid (C.I.-No. 77.492) | 1,500 |
| Polyester-3, Red 28 *) | 5,000 |
| Tocopherolacetat | 0,350 |
| Fragrance | 0,250 |

Die Herstellung von Beispiel 3 erfolgt in Analogie zu Beispiel 1. Diese Mascara-Zubereitung eignet sich besonders zum temporären Einfärben einzelner Strähnen und ergibt auf dem Haar eine orangegelbe Einfärbung, die unter langwelligem UV-Licht eine leuchtend rote Fluoreszenz zeigt.
*) "Polyester 3, Red 28" ist eine feste Lösung von D&C Red No. 28 (C.I.-No. 45410) in einer Polyester-Matrix.

### Beispiel 4 (pastenförmiger Effekt-Sonnenschutz für Surfer)

| | |
|---|---|
| Octamethyltrisiloxan | 40,900 |
| Dimethicone (MG 500.000) | 8,500 |
| Cera Microcristallina (81-84 °C) | 5,000 |
| C24-28 Alkyldimethicone | 5,500 |
| Bienenwachs, weiß | 2,500 |
| C30-38 Olefin/Isopropyl Maleate/MA Copolymer | 4,500 |
| C20-C40 Alcohols | 3,500 |
| Isoamyl p-Methoxycinnamate | 4,000 |
| 4-Methylbenzylidene Camphor | 3,500 |
| Buxus Chinensis (Jojobaöl) | 7,000 |
| Titandioxid (Nanopigment 10-20 nm) | 8,000 |
| Titandioxid (C.I.-No. 77.891) | 1,500 |
| Ultramarin (C.I.-No. 77.007) | 5,000 |
| Tocopherolacetat | 0,350 |
| Fragrance | 0,250 |

Die Herstellung von Beispiel 4 erfolgt analog zu Beispiel 1. Man erhält eine strahlend-blaue Paste mit der Wirkung eines Sunblockers. Wenn Farbvarianten erwünscht sind, kann das Ultramarin auch durch gelbes oder rotes Eisenoxid Chromoxidhydratgrün, Chromoxidgrün Manganviolett, Titandioxid oder deren Abmischungen ersetzt werden. Mittels eines Roll-on Applikators aufgetragen, lassen sich Linien oder Zeichnungen auf der Haut aufbringen.

### Beispiel 5 (pastenförmiger, farbloser Sunblocker)

| | |
|---|---|
| Octamethyltrisiloxan | 41,400 |
| Dimethicone (MG 500.000) | 9,500 |
| Cera Microcristallina (81-84 °C) | 7,000 |
| C24-28 Alkyldimethicone | 6,500 |
| Ouricurywachs | 4,500 |
| C30-38 Olefin/Isopropyl Maleate/MA Copolymer | 4,500 |
| C20-C40 Alcohols | 3,500 |
| Isoamyl p-Methoxycinnamate | 5,500 |
| 4-Methylbenzylidene Camphor | 4,500 |
| Buxus Chinensis (Jojobaöl) | 3,000 |
| Titandioxid (Nanopigment 10-20 nm) | 8,000 |
| Quaternium-18 Hectorite | 1,500 |
| Tocopherolacetat | 0,350 |
| Fragrance | 0,250 |

Die Herstellung von Beispiel 5 erfolgt analog zu Beispiel 1. Man erhält eine farblose, transluzente Paste, die zur Abfüllung in Tuben geeignet ist. Sie bietet eine hohen Lichtschutz im Bereich um SF 25-30.

### Beispiel 6 (pastenförmiger Lidschatten)

| | |
|---|---|
| Octamethylcyclotetrasiloxan | 35,000 |
| Decamethylcyclopentasiloxan | 14,000 |
| Dimethicone (MG 500.000) | 3,600 |
| Ouricurywachs | 6,200 |
| C20-C40 Alcohols | 4,800 |
| C30-38 Olefin/Isopropyl Maleate/MA Copolymer | 5,200 |
| Hydroxypropyldimethicone Behenate | 7,200 |
| Lauryldimethicone | 7,200 |
| Silica | 2,000 |
| Titanated Mica (C.I.-No. 77891) | 8,000 |
| Mica | 2,000 |
| Chromoxidhydratgrün (C.I.-No. 77289) | 3,200 |
| Ultramarin (C.I.-No. 77007) | 0,800 |
| Tocopherol | 0,600 |
| Fragrance | 0,200 |

Die Herstellung des Beispiels 6 erfolgt analog zu Beispiel 1, wobei aber die Pearlpigmente erst nach dem zweiten Passieren des Dreiwalzenstuhls zugesetzt werden. Die kräftig türkisfarbene Masse mit hellem Perlglanz eignet sich zur Abfüllung in kleine Tiegel oder, zur verbesserten Handhabung und Produkthygiene, ein längliches Behältnis mit einem an einem Stiel befestigten Applikator, einem sog. "Deerfoot-Applikator" als Halterung, die mit der Verschlußkappe verbunden ist.

### Beispiel 7 (fließfähige Foundation, wasserfest)

| | |
|---|---|
| Octamethylcyclotetrasiloxan | 34,200 |
| Decamethylcyclopentasiloxan | 42,700 |
| Dimethiconol Stearat | 0,850 |
| Bishydroxyethoxypropyldimethicone | 5,100 |
| Cetearyl Dimethicone/Vinyldimethicone | |
| Crosspolymer | 1,700 |
| Cera Microcristallina (81-84 °C) | 2,200 |
| Candelilla Cera | 0,900 |
| C20-28 Alkyldimethicone | 1,700 |
| C20-C40 Alcohols | 1,700 |
| C30-38 Olefin/Isopropyl Maleate/MA Copolymer | 1,700 |
| Tocopherol | 0,550 |
| Silica | 0,450 |
| Titandioxid (C.I.-No. 77891) | 3,500 |
| gelbes Eisenoxid (C.I.-No. 77492) | 1,200 |
| rotes Eisenoxid (C.I.-No. 77491) | 0,850 |
| schwarzes Eisenoxid (C.I.-No. 77499) | 0,450 |
| Fragrance | 0,250 |

Die Herstellung des Beispiels 7 erfolgt analog zu Beispiel 1. Man erhält eine hellbraune pastöse Zubereitung, die gut deckt und lange hält, mit guter Transferresistenz und die gegen Feuchtigkeit, z.B. Schweiß, sehr beständig ist. Sie eignet sich gut zur Abfüllung in Tuben.

### Beispiel 8 (fließfähiger Lipliner)

| | |
|---|---|
| Octamethylcyclotetrasiloxan | 68,000 |
| Cera Microcristallina | 6,800 |
| Candelilla Cera | 1,400 |
| Dimethicone (MG 500.000) | 4,100 |
| C24-28 Alkyldimethicone | 2,700 |
| C20-C40 Alcohols | 0,700 |
| Tocopherol | 0,500 |
| Octadecene/MA Copolymer | 0,700 |
| Diethyl Sebacate | 0,900 |
| Silica | 0,400 |
| Titandioxid (C.I.-No. 77891) | 4,100 |
| rotes Eisenoxid (C.I.-No. 77491) | 6,000 |
| FD&C Red No. 40 (C.I.-No. 16035) | 3,500 |
| Fragrance | 0,200 |

Die Herstellung des Beispiels 8 erfolgt analog zu Beispiel 1, wobei man, um eine Masse zu erhalten, die auf einem Dreiwalzenstuhl verarbeitbar ist, der heißen Masse etwa 20-25 % des flüchtigen Silikonöls zusetzt. Man erhält eine kräftig rote, gut deckende, noch fließende Zubereitung, die in kleine Behältnisse mittels einer in die Verschlußkappe eingesetzten Faserspitze aufgetragen werden kann. Die Zubereitung ist transferresistent und wandert nicht aus in die Feinfältelung der Haut.

### Beispiel 9 (fließfähiger Eyeliner)

| | |
|---|---|
| Octamethyltrisiloxan | 52,000 |
| Octamethylcyclotetrasiloxan | 20,000 |
| Cera Microcristallina | 5,000 |
| Rice Bran Wax | 2,000 |
| C20-C40 Alcohols | 1,600 |
| Dimethicone (MG 500.000) | 2,100 |
| C30-38 Olefin/Isopropyl Maleate/MA Copolymer | 1,800 |
| Phenylpropylpolysilsesquisiloxane | 2,400 |
| Tocopherol | 0,400 |
| Silica | 0,500 |
| schwarzes Eisenoxid (C.I.-No. 77499) | 10,000 |
| Ultramarin (C.I.-No. 77007) | 1,300 |
| Carmin (C.I.-No. 75470) | 0,700 |
| Fragrance | 0,200 |

Die Herstellung des Beispiels 9 erfolgt analog zu Beispiel 8. Man erhält eine fließfähige Zubereitung mit tiefschwarzer Farbe und mit sehr guter Haltbarkeit und Transferresistenz. Zur einfachen Handhabung erfolgt sie Abfüllung in die zu Beispiel 8 erwähnten Behältnisse.

### Beispiel 10 (pastöse Abdeckcreme bei Couperose, wasserbeständig)

| | |
|---|---|
| Octamethyltrisiloxan | 9,200 |
| Octamethylcyclotetrasiloxan | 46,000 |
| Decamethylcyclopentasiloxan | 15,200 |
| Cera Microcristallina | 3,800 |
| C24-28 Alkyldimethicone | 5,700 |
| C20-C40 Alcohols | 1,900 |
| C30-C38 Olefin/Isopropyl Maleate/MA Copolymer | 1,900 |
| Methoxy Amodimethicone Silsesquioxane Copolymer | 1,900 |
| Dimethicone (MG 500.000) | 4,700 |
| Tocopherol | 0,450 |
| Mica | 1,000 |
| Titandioxid (C.I.-No. 77891) | 4,500 |
| Chromoxidhydratgrün (C.I.-No. 77269) | 3,500 |
| Fragrance | 0,250 |

Die Herstellung des Beispiels 10 erfolgt analog zu Beispiel 1. Man erhält eine helltürkisfarbene Zubereitung mit guter Deckkraft und sehr guter Transferresistenz, die sich leicht und gleichmäßig auf der Haut verteilen läßt. Die Zubereitung ist zur Abfüllung in Tuben geeignet. Sie dient dazu, Hautanomalien wie Pigmentflecken und durchscheinende Äderchen (Couperose und Rosacea) aufgrund ihrer Komplementärfarbe zu kaschieren. Da die Zubereitung wasserbeständig ist und von O/W-Emulsionen nicht angelöst wird, kann nach dem Trocknen nach dem Geschmack der jeweiligen Trägerin ein passendes Make-up aufgetragen werden, ohne dass sich die Schichten vermischen oder beeinträchtigen.

## Patentansprüche

1. Wasserfreie Zubereitung, insbesondere kosmetische Zubereitung, in flüssiger oder pastöser Form, die eine Wachskomponente und als Solubilisierungsmittel eine flüssige Silikonverbindung enthält, wobei die Wachskomponente aus mindestens einem Wachs mit einem Tropfpunkt oberhalb von 60°C und einem Kompatibilisierungsmittel, mindestens einem nicht flüchtigen ÖI und/oder einem langkettigen Alkohol und/oder einem filmbildenden Polymer oder Copolymer mit einem Tropfpunkt von mindestens 30°C gebildet wird, wobei das Kompatibilisierungsmittel ausgewählt ist aus Lauryldimethicon, Cetyldimethicon, Cetearyldimethicon, Behenyl-dimethicon, C24-28 Alkyldimethicon, Bishydroxyethoxypropyldimethicon, Phenylpropylsilsesquisiloxan, Dimethiconolstearat und/oder Hydroxypropyldimethicon-behenat und Mischungen daraus.

2. Zubereitung nach Anspruch 1, **dadurch gekennzeichnet, dass** sie frei ist von nichtflüchtigen Mineralölen und von flüssigen oder bei Raumtemperatur festen Triglyceriden.

3. Zubereitung nach Anspruch 1, **dadurch gekennzeichnet, dass** das Wachs eine Nadelpenetration nach ASTM D5 von 2 bis 40 aufweist, bestimmt bei einer Temperatur von 25 °C und einer Eindringzeit von 5 sec, bei einem Gesamtgewicht des Prüfkörpers von 50 g.

4. Zubereitung nach einem der vorhergehenden Ansprüche **dadurch gekennzeichnet, dass** das Wachs ausgewählt ist unter Bienenwachs, modifiziertem Bienenwachs , Candelillawachs, Carnaubawachs, Ouricuriwachs, Reiswachs, Montanwachs, mikrokristallines Wachs, Paraffinwachs, Ozokerit, Polyethylenwachs, nach dem Fischer-Tropsch-Verfahren hergestelltes Wachs, Silikonwachs oder deren Mischungen.

5. Zubereitung nach einem der vorhergehenden Ansprüche, **dadurch gekennzeichnet, dass** die Zubereitung weitere Wachse und/oder langkettige Ester enthält, die ausgewählt sind aus Lanolinwachs, Japanwachs, Orangenblütenwachs, Orangenwachs, Cetylpalmitat, Stearylstearat, Behenylstearat, Behenylbehenat, hydriertem Jojobaöl oder Mischungen daraus und/oder ihren Hybriden.

6. Zubereitung nach einem der vorhergehenden Ansprüche **dadurch gekennzeichnet, dass** die Einsatzmenge der Wachse, ihrer Mischungen und oder ihrer Hybride im Bereich zwischen 3 und 35 Gew.-%, bevorzugt zwischen 5 und 25 Gew.-%, besonders bevorzugt zwischen 8 und 20 Gew.-% liegt.

7. Zubereitung nach einem der vorhergehenden Ansprüche, **dadurch gekennzeichnet, dass** die flüssige Silikonverbindung ausgewählt ist aus linearen, verzweigten oder cyclischen Silikonölen oder deren Mischungen.

8. Zubereitung nach Anspruch 7, **dadurch gekennzeichnet, dass** das Silikonöl ausgewählt ist aus Hexamethyldisiloxan, Octamethyltrisiloxan, Decamethyltetrasiloxan, Dodecamethylpentasiloxan, Octamethylcyclotetrasiloxan, Decamethylcyclopentasiloxan, Dodecamethylcyclohexasiloxan oder Mischungen daraus.

9. Zubereitung nach Anspruch 7 oder 8, **dadurch gekennzeichnet, dass** die Einsatzmenge der flüssigen Silikonverbindung oder des Kohlenwasserstofföls zwischen 10 und 80 Gew.-%, bevorzugt zwischen 15 und 65 Gew.% und besonders bevorzugt zwischen 25 und 50 Gew.-% liegt.

10. Zubereitung nach einem der vorhergehenden Ansprüche **dadurch gekennzeichnet, dass** die Wachskomponente ein nichtflüchtiges ÖI ausgewählt aus hochmolekularen Verbindungen auf Basis von Kohlenwasserstoff- oder Siloxaneinheiten, mittel- und langkettigen Carbonsäureestern oder Mischungen daraus enthält.

11. Zubereitung nach Anspruch 10, **dadurch gekennzeichnet, dass** das nichtflüchtige ÖI ausgewählt ist aus hochmolekularen Dimethylpolysiloxanen (Dimethicone), Phenyltrimethicone, Diphenyldimethicone oder Mischungen daraus.

12. Zubereitung nach Anspruch 11, **dadurch gekennzeichnet, dass** das Dimethylpolysiloxan ausgewählt ist aus den langkettigen linearen Dimethylpolysiloxanen mit einem Molekulargewicht von mehr als 100.000 Dalton, bevorzugt mehr als 250.000 Dalton, ganz besonders bevorzugt mehr als 450.000 Dalton.

13. Zubereitung nach Anspruch 10, **dadurch gekennzeichnet, dass** die mittel- und langkettigen Carbonsäureester ausgewählt sind aus Jojobaöl, Oleylerucat, Oleyloleat, Hexyllaurat, Dibutyladipat, Diethylsebacat, Isostearyl-isostearat oder Mischungen daraus.

14. Zubereitung nach einem der vorhergehenden Ansprüche **dadurch gekennzeichnet, dass** die Einsatzmenge der nichtflüchtigen ÖIe zwischen 1 und 30 Gew.% und bevorzugt zwischen 2 und 25 Gew.% liegt, bezogen auf die Zubereitung.

15. Zubereitung nach einem der vorhergehenden Ansprüche, **dadurch gekennzeichnet, dass** die Einsatzmenge an Alkyldimethicone zwischen 0,5 und 35 Gew.-%, bevorzugt zwischen 1,5 und 25 Gew.-% und ganz besonders bevorzugt zwischen 3 und 12 Gew.-% liegt.

16. Zubereitung nach einem der vorhergehenden Ansprüche **dadurch gekennzeichnet, dass** mindestens ein bei Raumtemperatur fester, langkettiger Alkohol enthalten ist ausgewählt aus gesättigten geradkettigen oder verzweigtkettigen Alkoholen eine Kettenlänge zwischen 14 und 48 Kohlenstoffatomen oder Mischungen daraus.

17. Zubereitung nach Anspruch 16, **dadurch gekennzeichnet, dass** der bei Raumtemperatur feste, langkettige Alkohol ausgewählt ist aus Myristylalkohol, Cetylalkohol, Stearylalkohol, Behenylalkohol, Cerylalkohol, Myricylalkohol oder Mischungen daraus.

18. Zubereitung nach Anspruch 16 oder 17, **dadurch gekennzeichnet, dass** die Einsatzmenge der Alkohole zwischen 0,5 und 20 Gew.-%, bevorzugt zwischen 2,5 und 15 Gew.-%, besonders bevorzugt zwischen 4 und 12 Gew.-% liegt.

19. Zubereitung nach einem der vorhergehenden Ansprüche **dadurch gekennzeichnet, dass** sie ein filmbildendes flexibles Polymer oder Copolymer enthält, das einen Tropfpunkt von mindestens 30 °C aufweist.

20. Zubereitung nach Anspruch 19, **dadurch gekennzeichnet, dass** das filmbildende flexible Polymer oder Copolymer ausgewählt ist aus C30-38 Olefin/Isopropyl Maleate/ MA Copolymer, Cetearyl DimethiconeNinyldimethicone Crosspolymer, Octadecene/MA Copolymer, Methoxy Amodimethicone Silsesquioxane Copolymer oder Mischungen daraus.

21. Zubereitung nach Anspruch 19 oder 20, **dadurch gekennzeichnet, dass** die Einsatzmenge des Polymers oder Copolymers zwischen 0,1 und 20 Gew.-%, bevorzugt zwischen 0,5 und 12 Gew.-% und ganz besonders bevorzugt zwischen 2 und 8 Gew.-% liegt.

22. Zubereitung nach einem der vorhergehenden Ansprüche **dadurch gekennzeichnet, dass** die Zubereitung in Form von Make-up, Concealer, Camouflage, Lidschatten, Eyeliner, Lipliner, Rouge, Lippenrouge, Lipgloss, Sonnenschutzmittel, Sunblocker, temporäres Tattoo, Mascara, Haar-Mascara, Effekt-Sonnenschutz für Surfer und als Abdeckcreme bei Couperose und Rosacea vorliegt.

## Claims

1. A water-free preparation, in particular a cosmetic preparation, in liquid or pasty form, that contains a wax component and, as a solubilization agent, a liquid silicone compound, wherein the wax component is formed from at least one wax with a dropping point above 60°C and a compatibilization agent, at least one non-volatile oil and/or a long-chain alcohol and/or a film-forming polymer or copolymer with a dropping point of at least 30°C, wherein the compatibilization agent is selected from lauryl dimethicone, cetyl dimethicone, cetearyl dimethicone, behenyl dimethicone, C24-28 alkyl dimethicone, bishydroxyethoxy propyl dimethicone, phenyl propyl silsesquisiloxane, dimethiconol stearate and/or hydroxypropyl dimethicone behenate and mixtures thereof.

2. A preparation according to claim 1, **characterised in that** it is free of non-volatile mineral oils and of triglycerides that are liquid or solid at ambient temperature.

3. A preparation according to claim 1, **characterised in that** the wax has a needle penetration in accordance with ASTM D5 of 2 to 40, determined at a temperature of 25°C and with a penetration time of 5 sec, with a total weight of the test body of 50 g.

4. A preparation according to one of the preceding claims, **characterised in that** the wax is selected from beeswax, modified beeswax, candelilla wax, carnauba wax, ouricuri wax, rice wax, montan wax, microcrystalline wax, paraffin wax, ozocerite, polyethylene wax, wax produced in accordance with the Fischer-Tropsch process, silicone wax or mixtures thereof.

5. A preparation according to one of the preceding claims, **characterised in that** the preparation contains further waxes and/or long-chain esters which are selected from lanolin wax, Japan wax, orange flower wax, orange wax, cetyl palmitate, stearyl stearate, behenyl stearate, behenyl behenate, hydrogenated jojoba oil or mixtures thereof and/or their hybrids.

6. A preparation according to one of the preceding claims, **characterised in that** the amount of the waxes, their mixtures and/or their hybrids used is in the range between 3 and 35% by weight, preferably between 5 and 25% by weight, particularly preferably between 8 and 20% by weight.

7. A preparation according to one of the preceding claims, **characterised in that** the liquid silicone compound is selected from linear, branched or cyclic silicone oils or mixtures thereof.

8. A preparation according to claim 7, **characterised in that** the silicone oil is selected from hexamethyl disiloxane, octamethyl trisiloxane, decamethyl tetrasiloxane, dodecamethyl pentasiloxane, octamethyl cyclotetrasiloxane, decamethyl cyclopentasiloxane, dodecamethyl cyclohexasiloxane or mixtures thereof.

9. A preparation according to claim 7 or 8, **characterised in that** the amount of the liquid silicone compound or hydrocarbon oil used is between 10 and 80% by weight, preferably between 15 and 65% by weight and particularly preferably between 25 and 50% by weight.

10. A preparation according to one of the preceding claims, **characterised in that** the wax component contains a non-volatile oil selected from high-molecular compounds based on hydrocarbon or siloxane units, medium- and long-chain carboxylic acid esters or mixtures thereof.

11. A preparation according to claim 10, **characterised in that** the non-volatile oil is selected from high-molecular dimethyl polysiloxane (dimethicone), phenyl trimethicone, diphenyl dimethicone or mixtures thereof.

12. A preparation according to claim 11, **characterised in that** the dimethyl polysiloxane is selected from the long-chain linear dimethyl polysiloxanes of a molecular weight above 100,000 daltons, preferably above 250,000 daltons, especially preferably above 450,000 daltons.

13. A preparation according to claim 10, **characterised in that** the medium- and long-chain carboxylic acid esters are selected from jojoba oil, oleyl erucate, oleyl oleate, hexyl laurate, dibutyl adipate, diethyl sebacate, isostearyl isostearate or mixtures thereof.

14. A preparation according to one of the preceding claims, **characterised in that** the amount of the non-volatile oils used is between 1 and 30% by weight and preferably between 2 and 25% by weight, in relation to the preparation.

15. A preparation according to one of the preceding claims, **characterised in that** the amount of alkyl dimethicone used is between 0.5 and 35% by weight, preferably between 1.5 and 25% by weight and especially preferably between 3 and 12% by weight.

16. A preparation according to one of the preceding claims, **characterised in that** there is contained at least one long-chain alcohol that is solid at ambient temperature and selected from saturated straight-chain or branched-chain alcohols of a chain length of between 14 and 48 carbon atoms or mixtures thereof.

17. A preparation according to claim 16, **characterised in that** the long-chain alcohol that is solid at ambient temperature is selected from myristyl alcohol, cetyl alcohol, stearyl alcohol, behenyl alcohol, ceryl alcohol, myricyl alcohol or mixtures thereof.

18. A preparation according to claim 16 or 17, **characterised in that** the amount of the alcohols used is between 0.5 and 20% by weight, preferably between 2.5 and 15% by weight, particularly preferably between 4 and 12% by weight.

19. A preparation according to one of the preceding claims, **characterised in that** it contains a film-forming, flexible polymer or copolymer that has a dropping point of at least 30°C.

20. A preparation according to claim 19, **characterised in that** the film-forming, flexible polymer or copolymer is selected from C30-38 olefin/isopropyl maleate/MA copolymer, cetearyl dimethicone/vinyl dimethicone cross-polymer, octadecene/MA copolymer, methoxy amodimethicone silsesquioxane copolymer or mixtures thereof.

21. A preparation according to claim 19 or 20, **characterised in that** the amount of the polymer or copolymer used is between 0.1 and 20% by weight, preferably between 0.5 and 12% by weight and especially preferably between 2 and 8% by weight.

22. A preparation according to one of the preceding claims, **characterised in that** the preparation is in the form of make-up, concealer, camouflage, eye shadow, eye liner, lip liner, rouge, lip rouge, lip gloss, sun-screen agent, sun block, temporary tattoo, mascara, hair mascara, effect sun-protection for surfers and as covering cream in relation to couperose and rosacea.

## Revendications

1. Préparation anhydre, en particulier préparation cosmétique, sous forme fluide ou pâteuse, qui contient un composant de cire et en tant qu'agent de solubilisation un composé de silicone liquide, dans laquelle le composant de cire est formé d'au moins une cire présentant un point de goutte supérieur à 60 °C et d'un agent de compatibilité, d'au moins une huile non volatile et/ou un alcool à chaîne longue et/ou un polymère ou copolymère filmogène présentant un point de goutte au moins égal à 30 °C, dans laquelle l'agent de compatibilité est sélectionné parmi la lauryldiméthicone, la cétyldiméthicone, la cétéaryldiméthicone, la béhényl-diméthicone, une diméthicone d'alkyle en C24-28, la bishydroxyéthoxypropyldiméthicone, le phénylpropylsilsesquisiloxane, le stéarate de diméthiconol et/ou l'hydroxypropylbéhénate de diméthicone et des mélanges de ceux-ci.

2. Préparation selon la revendication 1, **caractérisée en ce qu'**elle est exempte d'huiles minérales non volatiles et de triglycérides liquides ou solides à la température ambiante.

3. Préparation selon la revendication 1, **caractérisée en ce que** la cire présente une pénétrabilité à l'aiguille selon ASTM D5 dans la plage de 2 à 40, déterminée à une température de 25 °C et une durée de pénétration de 5 secondes, pour un poids total de l'éprouvette de 50 g.

4. Préparation selon l'une des revendications précédentes, **caractérisée en ce que** la cire est sélectionnée parmi de la cire d'abeilles, de la cire d'abeilles modifiée, de la cire de candellila, de la cire de carnauba, de la cire d'ouricuri, de la cire de riz, de la cire de lignite, de la cire microcristalline, de la cire de paraffine, de l'ozokérite, de la cire de polyéthylène, de la cire fabriquée selon le procédé de Fischer-Tropsch, de la cire de silicone ou des mélanges de celles-ci.

5. Préparation selon l'une des revendications précédentes, **caractérisée en ce que** la préparation contient d'autres cires et/ou esters à chaîne longue qui sont sélectionnés parmi de la cire de lanoline, de la cire du Japon, de la cire de fleur d'oranger, de la cire d'orange, du palmitate de cétyle, du stéarate de stéaryle, du stéarate de béhényle, du béhénate de béhényle, de l'huile de jojoba hydrogénée ou leurs mélanges et/ou hybrides.

6. Préparation selon l'une des revendications précédentes, **caractérisée en ce que** la quantité utilisée des cires, de leurs mélanges et/ou de leurs hybrides, est comprise dans la plage de 3 à 35 % en poids, de préférence de 5 à 25 % en poids, et de façon particulièrement préférée de 8 à 20 % en poids.

7. Préparation selon l'une des revendications précédentes, **caractérisée en ce que** le composé de silicone liquide est sélectionné parmi des huiles de silicone linéaires, ramifiées ou cycliques, ou des mélanges de celles-ci.

8. Préparation selon la revendication 7, **caractérisée en ce que** l'huile de silicone est sélectionnée parmi l'hexaméthyldisiloxane, l'octaméthyltrisiloxane, le décaméthyltétrasiloxane, le dodécaméthylpentasiloxane, l'octaméthylcyclotétrasiloxane, le décaméthylcyclopentasiloxane, le dodécaméthylcyclohexasiloxane, ou des mélanges de ceux-ci.

9. Préparation selon la revendication 7 ou 8, **caractérisée en ce que** la quantité utilisée du composé de silicone liquide ou de l'huile d'hydrocarbure est comprise entre 10 à 80 % en poids, de préférence entre 15 et 65 % en poids et de façon particulièrement préférée entre 25 et 50 % en poids.

10. Préparation selon l'une des revendications précédentes, **caractérisée en ce que** le composant de cire contient une huile non volatile sélectionnée parmi des composés de poids moléculaire élevé à base de motifs hydrocarbure ou siloxane, des esters d'acide carboxylique à chaîne moyenne et longue, ou des mélanges de ceux-ci.

11. Préparation selon la revendication 10, **caractérisée en ce que** l'huile non volatile est sélectionnée parmi des diméthylpolysiloxanes de poids moléculaire élevé (diméthicones), des phényltriméthicones, des diphényldiméthicones ou des mélanges de ceux-ci.

12. Préparation selon la revendication 11, **caractérisée en ce que** le diméthylpolysiloxane est sélectionné parmi les diméthylpolysiloxanes linéaires à chaîne longue présentant un poids moléculaire de plus de 100 000 daltons, de préférence de plus de 250 000 daltons, de façon particulièrement préférée de plus de 450 000 daltons.

13. Préparation selon la revendication 10, **caractérisée en ce que** les esters d'acide carboxylique à chaîne moyenne et longue sont sélectionnés parmi de l'huile de jojoba, de l'érucate d'oléyle, de l'oléate d'oléyle, du laurate d'hexyle, de l'adipate de dibutyle, du sébacate de diéthyle, de l'isostéarate d'isostéaryle ou des mélanges de ceux-ci.

14. Préparation selon l'une des revendications précédentes, **caractérisée en ce que** la quantité utilisée d'huiles non volatiles est de 1 à 30 % en poids, et de préférence de 2 à 25 % en poids, sur la base de la préparation.

15. Préparation selon l'une des revendications précédentes, **caractérisée en ce que** la quantité utilisée d'alkyldiméthicone est de 0,5 à 35 % en poids, de préférence de 1,5 à 25 % en poids, et de façon particulièrement préférée de 3 à 12 % en poids.

16. Préparation selon l'une des revendications précédentes, **caractérisée en ce qu'**est contenu au moins un alcool à chaîne longue solide à la température ambiante, sélectionné parmi des alcools saturés à chaîne linéaire ou ramifiée comportant une longueur de chaîne de 14 à 48 atomes de carbone, ou des mélanges de ceux-ci.

17. Préparation selon la revendication 16, **caractérisée en ce que** l'alcool à chaîne longue solide à la température ambiante est sélectionné parmi l'alcool de myristyle, l'alcool de cétyle, l'alcool de stéaryle, l'alcool de béhényle, l'alcool de céryle, l'alcool de myricyle ou des mélanges de ceux-ci.

18. Préparation selon la revendication 16 ou 17, **caractérisée en ce que** la quantité utilisée des alcools est de 0,5 à 20 % en poids, de préférence de 2,5 à 15 % en poids, et de façon particulièrement préférée de 4 à 12 % en poids.

19. Préparation selon l'une des revendications précédentes, **caractérisée en ce qu'**elle présente un polymère ou copolymère filmogène flexible qui présente un point de goutte au moins égal à 30 °C.

20. Préparation selon la revendication 19, **caractérisée en ce que** le polymère ou copolymère filmogène flexible est sélectionné parmi un copolymère d'oléfine en C30-38 / maléate d'isopropyle / MA, un polymère réticulé de cétéaryldiméthicone / vinyldiméthicone, un copolymère d'octadécène / MA, un copolymère de méthoxy-amodiméthicone silsesquioxane ou des mélanges de ceux-ci.

21. Préparation selon la revendication 19 ou 20, **caractérisée en ce que** la quantité utilisée du polymère ou du copolymère est de 0,1 à 20 % en poids, de préférence de 0,5 à 12 % en poids, et de façon particulièrement préférée de 2 à 8 % en poids.

22. Préparation selon l'une des revendications précédentes, **caractérisée en ce que** la préparation se présente sous la forme d'un maquillage, d'un cache-cernes, d'un produit correcteur, d'une ombre à paupières, d'un eye-liner, d'un crayon à lèvres, d'un rouge, d'un rouge à lèvres, d'un brillant à lèvres, d'un agent de protection solaire, d'un écran solaire, d'un tatouage temporaire, d'un mascara, d'un mascara capillaire, d'une protection solaire à effet pour surfeurs et d'une crème de masquage de la couperose et de la rosacée.
